(19) Europäisches Patentamt
European Patent Office
Office européen des brevets

(11) EP 4 339 609 A1

(12) EUROPEAN PATENT APPLICATION
published in accordance with Art. 153(4) EPC

(43) Date of publication:
**20.03.2024 Bulletin 2024/12**

(21) Application number: **23796293.1**

(22) Date of filing: **21.04.2023**

(51) International Patent Classification (IPC):
***G01N 33/00*** *(2006.01)* ***C10L 1/02*** *(2006.01)*
***C10L 1/08*** *(2006.01)* ***F02D 45/00*** *(2006.01)*
***G01N 25/18*** *(2006.01)* ***G01N 27/22*** *(2006.01)*
***G01N 33/22*** *(2006.01)*

(52) Cooperative Patent Classification (CPC):
**C10L 1/02; C10L 1/08; F02D 45/00; G01N 25/18; G01N 27/22; G01N 33/00; G01N 33/22**

(86) International application number:
**PCT/JP2023/016028**

(87) International publication number:
**WO 2023/210553 (02.11.2023 Gazette 2023/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.04.2022 JP 2022073900**

(71) Applicant: **Sun-a Corporation**
**Miyoshi-shi, Hiroshima 728-0017 (JP)**

(72) Inventors:
- **YAMAGISHI Kiyoshi**
  **Miyoshi-shi, Hiroshima 728-0017 (JP)**
- **ISHIBASHI Nobuhiro**
  **Miyoshi-shi, Hiroshima 728-0017 (JP)**
- **SATO Masaharu**
  **Miyoshi-shi, Hiroshima 728-0017 (JP)**
- **YANAGI Kiyotaka**
  **Miyoshi-shi, Hiroshima 728-0017 (JP)**
- **HABATA Keigo**
  **Miyoshi-shi, Hiroshima 728-0017 (JP)**
- **SUNADA Hiroaki**
  **Miyoshi-shi, Hiroshima 728-0017 (JP)**
- **HATONO Atsuo**
  **Miyoshi-shi, Hiroshima 728-0017 (JP)**
- **HIRUTA Tatsuya**
  **Miyoshi-shi, Hiroshima 728-0017 (JP)**
- **TATEDA Kazuya**
  **Miyoshi-shi, Hiroshima 728-0017 (JP)**

(74) Representative: **Hoffmann Eitle**
**Patent- und Rechtsanwälte PartmbB**
**Arabellastraße 30**
**81925 München (DE)**

# (54) COMPOSITION IDENTIFICATION PROGRAM, COMPOSITION IDENTIFICATION DEVICE, AND COMPOSITION IDENTIFICATION SYSTEM

(57) Provided is a composition identification program, a composition identification apparatus, and a composition identification system capable of identifying the composition of a mixture including a plurality of components.

A composition identification program for identifying a composition of a mixture of three different liquids of a liquid A, a liquid B, and a liquid C, the composition identification program being configured to make a computer apparatus function as a composition identifier that identifies a composition of the mixture on a basis of a first characteristic value and a second characteristic value of the mixture, a first characteristic value and a second characteristic value unique to the liquid A, a first characteristic value and a second characteristic value unique to the liquid B, and a first characteristic value and a second characteristic value unique to the liquid C.

EP 4 339 609 A1

FIG. 3

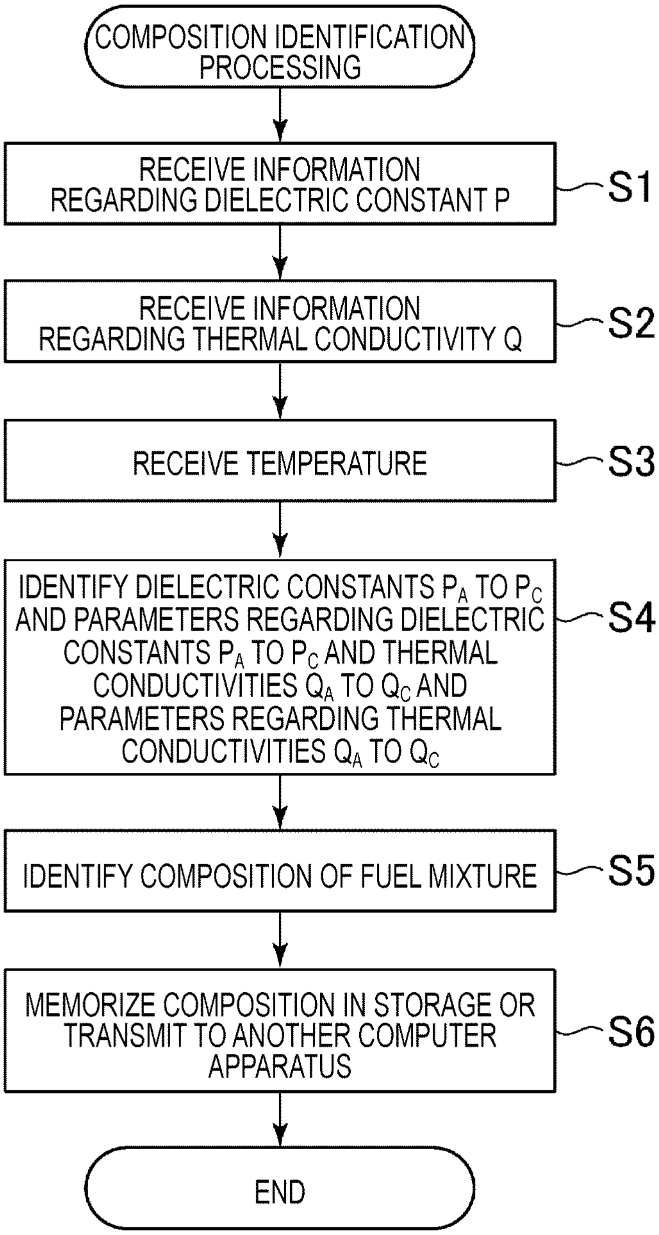
COMPOSITION IDENTIFICATION PROCESSING
RECEIVE INFORMATION REGARDING DIELECTRIC CONSTANT P
S1
RECEIVE INFORMATION REGARDING THERMAL CONDUCTIVITY Q
S2
RECEIVE TEMPERATURE
S3
IDENTIFY DIELECTRIC CONSTANTS $P_A$ TO $P_C$ AND PARAMETERS REGARDING DIELECTRIC CONSTANTS $P_A$ TO $P_C$ AND THERMAL CONDUCTIVITIES $Q_A$ TO $Q_C$ AND PARAMETERS REGARDING THERMAL CONDUCTIVITIES $Q_A$ TO $Q_C$
S4
IDENTIFY COMPOSITION OF FUEL MIXTURE
S5
MEMORIZE COMPOSITION IN STORAGE OR TRANSMIT TO ANOTHER COMPUTER APPARATUS
S6
END

## Description

Technical Field

The present invention relates to a composition identification program, a composition identification apparatus and a composition identification system.

Background Art

As measures against global warming, there is an increasing momentum for decarbonization. For example, unlike fossil fuel, bio-derived fuel or green fuel synthesized using green hydrogen and recovered carbon dioxide are considered to have zero carbon dioxide emissions even when these fuels are used.

Incidentally, as one of efforts to reduce carbon dioxide emissions, there is a credit system in which the reduction amount of carbon dioxide emissions and the absorption amount are certified by the government. According to the credit system, a business operator that has reduced carbon dioxide emissions can sell an authorized credit to another business operator. In a case where a mixture of a plurality of different fuels such as fossil fuel, bio-derived fuel, green fuel, and the like is used as fuel of a diesel vehicle, if the component ratio of the fuels can be identified, it is possible to identify the amount of carbon dioxide emission reduction caused by the operation of the diesel vehicle, and it is expected that the amount of carbon dioxide emission reduction can be used for credit certification.

In addition, when these fuels are added to already used fossil fuel, since the fuel properties such as kinematic viscosity, flow point, and cetane number are different, it is assumed that ideal parameters of engine control are different, and it is necessary to grasp the degree of mixing of the components and feed back to the engine control.

Summary of Invention

Technical Problem

An object of the present invention is to provide a composition identification program, a composition identification apparatus, and a composition identification system capable of identifying the composition of a mixture including a plurality of components.

Solution to Problem

According to the invention, the above objects are achieved by providing the following:

[1] A composition identification program for identifying a composition of a mixture of three different liquids of a liquid A, a liquid B, and a liquid C, the composition identification program being configured to make a computer apparatus function as a composition identifier that identifies a composition of the mixture on a basis of a first characteristic value and a second characteristic value of the mixture, a first characteristic value and a second characteristic value unique to the liquid A, a first characteristic value and a second characteristic value unique to the liquid B, and a first characteristic value and a second characteristic value unique to the liquid C;

[2] The composition identification program according to the [1], the composition identification program being configured to make a computer apparatus function as a characteristic value identifier that identifies the first characteristic value and/or the second characteristic value unique to the liquid A, the first characteristic value and/or the second characteristic value unique to the liquid B, and/or the first characteristic value and/or the second characteristic value unique to the liquid C according to a temperature of the mixture;

[3] The composition identification program according to the[1] or [2], wherein, in a case where the first characteristic value of the mixture is P and the second characteristic value of the mixture is Q, the first characteristic value of the liquid A is $P_A$ and the second characteristic value of the liquid A is $Q_A$, the first characteristic value of the liquid B is $P_B$, and the second characteristic value of the liquid B is $Q_B$, and the first characteristic value of the liquid C is Pc and the second characteristic value of the liquid C is Qc, the composition identifier identifies a volume fraction X vol% of the liquid A, a volume fraction Y vol% of the liquid B, and/or a volume fraction Z vol% of the liquid C on a basis of a formula:

[Mathematical formula 1]

$$X = 100 - Y - Z \quad \cdots (a)$$

$$Y = \frac{(P - P_A)(Q_C - Q_A) - (P_C - P_A)(Q - Q_A)}{(P_B - P_A)(Q_C - Q_A) - (P_C - P_A)(Q_B - Q_A)} \times 100 \quad \cdots (b)$$

$$Z = \frac{-(P - P_A)(Q_B - Q_A) + (P_B - P_A)(Q - Q_A)}{(P_B - P_A)(Q_C - Q_A) - (P_C - P_A)(Q_B - Q_A)} \times 100 \quad \cdots (c)$$

and identifies the composition of the mixture;

[4] The composition identification program according to any one of the [1] to [3], wherein the composition identifier identifies the composition of the mixture on a basis of a deviation amount between a volume fraction of the liquid A, the liquid B, and/or the liquid C calculated by a predetermined calculation formula and a volume fraction of the liquid A, the liquid B, and/or the liquid C in an actual mixture;

[5] The composition identification program according to any one of the [1] to [4], wherein the first characteristic value is a dielectric constant or a parameter regarding the dielectric constant, and the second characteristic value is a thermal conductivity or a parameter regarding the thermal conductivity;

[6] The composition identification program according to any one of the [1] to [5], wherein the liquid A is fossil fuel, the liquid B is hydrogenated vegetable oil, and the liquid C is bio-derived fuel;

[7] The composition identification program according to any one of the [1] to [6], the composition identification program being configured to make a computer apparatus function as a composition memory that memorizes the identified composition of the mixture and/or a composition transmitter that transmits the identified composition of the mixture to another computer apparatus;

[8] A composition identification apparatus for identifying a composition of a mixture of three different liquids of a liquid A, a liquid B, and a liquid C, the composition identification apparatus comprising a composition identifier that identifies a composition of the mixture on a basis of a first characteristic value and a second characteristic value of the mixture, a first characteristic value and a second characteristic value unique to the liquid A, a first characteristic value and a second characteristic value unique to the liquid B, and a first characteristic value and a second characteristic value unique to the liquid C;

[9] A composition identification apparatus for identifying a composition of a fuel mixture containing different types of fuels, the composition identification apparatus comprising a composition identifier that identifies a composition of the fuel mixture on a basis of one or a plurality of characteristic values of the fuel mixture and one or a plurality of characteristic values unique to each of a plurality of the fuels contained in the fuel mixture;

[10] The composition identification apparatus according to the [9], further comprising a composition memory that memorizes the identified composition of the fuel mixture and/or a composition transmitter that transmits the identified composition of the fuel mixture to another computer apparatus;

[11] The composition identification apparatus according to the [9] or [10], wherein the one or the plurality of characteristic values unique to each of the plurality of fuels are values identified according to a state of the fuel mixture or a surrounding environment of the fuel mixture;

[12] A composition identification system comprising: the composition identification apparatus according to any one of the [9] to [11]; a fuel unit in which a fuel mixture is present; and one or a plurality of measurers capable of measuring one or a plurality of different characteristic values of the fuel mixture present in the fuel unit, wherein the composition identifier identifies a composition of the fuel mixture on a basis of the one or the plurality of different characteristic values of the fuel mixture measured by the one or the plurality of measurers;

[13] A moving object comprising: the composition identification apparatus according to any one of the [9] to [12]; a fuel unit in which a fuel mixture is present; one or a plurality of measurers capable of measuring one or a plurality of different characteristic values of the fuel mixture present in the fuel unit; and a heat engine, wherein the composition identifier identifies a composition of the fuel mixture on a basis of the one or the plurality of different characteristic values of the fuel mixture measured by the one or the plurality of measurers, and wherein the moving object is driven by burning fuel supplied from the fuel unit in the heat engine.

## Advantageous Effects of Invention

The invention can provide a composition identification program, a composition identification apparatus, a composition identification system and moving object capable of identifying the composition of a mixture including a plurality of components.

## Brief Description of Drawings

Fig. 1 is a block diagram illustrating a configuration of a composition identification system according to an embodiment of the present invention.
Fig. 2 is a block diagram illustrating a configuration of a composition identification apparatus according to an embodiment of the present invention.
Fig. 3 is a flowchart illustrating composition identification processing according to an embodiment of the present invention.
Fig. 4 is a diagram illustrating a relationship between the dielectric constant and the temperature of three types of fuels.
Fig. 5 is a diagram illustrating a relationship between the dielectric constant and the thermal conductivity of three types of fuels.
Fig. 6 is a diagram illustrating a composition identification apparatus according to an embodiment of the present invention.
Fig. 7 is a diagram illustrating a relationship between the dielectric constant and the thermal conductivity of three types of fuels.
Fig. 8 is a diagram illustrating a relationship between the dielectric constant and the thermal conductivity of three types of fuels.
Fig. 9 is a graph in which, for a fuel mixture including two components, a deviation amount of an actual proportion of HVO or light oil in the fuel mixture from a calculated proportion of HVO or light oil is plotted on the vertical axis, and a proportion of BDF (registered trademark) in the fuel mixture is plotted on the horizontal axis.
Fig. 10 is a diagram illustrating a thermal conductivity sensor according to an embodiment of the present invention.

## Description of Embodiments

Hereinafter, embodiments of the invention will be described. The invention, however, is not limited to the following embodiments without departing from the spirit of the invention.

Fig. 1 is a block diagram illustrating a configuration of a composition identification system according to an embodiment of the present invention. A composition identification system 1 includes at least a composition identification apparatus 2 for identifying a composition of a mixture of a fuel including a plurality of different types of fuels (hereinafter, also referred to as a "fuel mixture") and a fuel unit 3 in which the fuel mixture is present. The fuel unit 3 is provided with a fuel tank that stores a fuel mixture. As the fuel, only one type of fuel may be stored or a fuel mixture including a plurality of different types of fuels may be stored.

The fuel unit 3 is provided with one or a plurality of measurers capable of measuring one or a plurality of characteristic values of the fuel mixture. The fuel unit 3 is a concept including not only a fuel tank that stores a fuel mixture but also a fuel supply line for supplying the fuel mixture to a heat engine 4 such as an engine or the like and an oil supply line for supplying the fuel mixture to the fuel tank, and it is sufficient that the one or a plurality of measurers capable of measuring one or a plurality of characteristic values of a fuel mixture are provided in any of the fuel tank, the fuel supply line, and the oil supply line.

The plurality of measurers can respectively measure different characteristic values. For example, one of the plurality of measurers can measure a parameter regarding a dielectric constant of the fuel mixture and another measurer can measure a parameter regarding a thermal conductivity of the fuel mixture. The measurers are not particularly limited as long as the characteristic value of the fuel mixture can be measured, and examples thereof include a sensor or a measuring instrument used for measuring a dielectric constant, a thermal conductivity, a specific gravity, a flash point, a viscosity, and the like. In the present invention, characteristic values for a plurality of characteristics (for example, dielectric constant, thermal conductivity, specific gravity, flash point, viscosity) of a fuel mixture are measured, and a composition of the fuel mixture is identified on the basis of the characteristic values. However, a type of the characteristic used to identify the composition of the fuel mixture is not particularly limited, and a combination of any type of characteristics to identify the composition of the fuel mixture is not particularly limited.

The characteristic value of a fuel mixture measured by the measurers is transmitted to the composition identification apparatus 2 by wired or wireless communication. The composition identification apparatus 2 can identify the composition of the fuel mixture on the basis of the received characteristic value of the fuel mixture.

When two types of fuels are contained in a fuel mixture, the composition of the fuel mixture can be identified on the basis of the characteristic value of the fuel mixture measured by one measurer and the characteristic value that is the same kind of characteristic value as the characteristic value of the fuel mixture measured by one measurer and is unique to each of the two types of fuels contained in the fuel mixture. In addition, when three types of fuels are contained in a fuel mixture, the composition of the fuel mixture can be identified on the basis of two different characteristic values of the fuel mixture measured by two different measurers and characteristic values that are the same kind of characteristic values as the two different characteristic values and are unique to each of the three types of fuels contained in the fuel mixture. Furthermore, when four types of fuels are contained in a fuel mixture, the composition of the fuel mixture can be identified on the basis of three different characteristic values of the fuel mixture measured by three different measurers, and characteristic values that are the same kind of characteristic values as the three different characteristic values and are unique to each of the four types of fuels contained in the fuel mixture.

In this manner, the number of types of characteristic values required to identify the composition of a fuel mixture also varies depending on the number of types of fuels contained in the fuel mixture. Specifically, when the number of types of fuels contained in a fuel mixture is defined as n, the number of types of characteristic values required to identify the composition of the fuel mixture is (n - 1) (n is an integer of 2 or more). That is, in the present invention, the composition of the mixture can be identified on the basis of a first characteristic value to an (n - 1)th characteristic value of the mixture of n types of different liquids and the first characteristic value to the (n - 1)th characteristic value unique to each liquid contained in the mixture.

The heat engine 4 may be either an internal combustion engine or an external combustion engine, and examples thereof include an internal combustion engine of a moving object 5 such as a vehicle, a ship, an aircraft, and the like. The vehicles include not only automobiles and motorcycles but also special vehicles such as construction vehicles, industrial vehicles, and the like. The composition identification apparatus 2 may be provided in the moving object 5 such as a vehicle, a ship, an aircraft, and the like together with the heat engine 4, or may be provided outside the moving object 5.

When the composition identification apparatus 2 is provided in the moving object 5 of a vehicle, a ship, or an aircraft together with the fuel unit 3 and the heat engine 4, a composition of a fuel mixture identified by the composition identification apparatus 2 or the information regarding the composition may be memorized in the composition identification apparatus 2, or may be transmitted from the composition identification apparatus 2 to another computer apparatus 7 via a communication network 6 by wired or wireless communication. Another computer apparatus 7 is provided inside or outside the moving object 5 provided with the composition identification apparatus 2. In another computer apparatus 7, the received a composition of the fuel mixture or an information regarding the composition can be memorized in a storage. Here, the information regarding the composition is a concept including information that can be calculated or identified from the composition, such as information indicating that the content of the fuel mixture of at least one liquid selected from a plurality of liquids contained in a fuel mixture or the content of a fuel mixture of the at least one liquid is equal to or more than a predetermined threshold value, is greater than the threshold value, is equal to or less than the threshold value, or is less than the threshold value. The same applies hereinafter.

Unlike Fig. 1, in a case where the composition identification apparatus 2 is provided outside the moving object 5, the characteristic value of a fuel mixture measured by the measurers is transmitted to the composition identification apparatus 2 by wireless communication. The composition identification apparatus 2 identifies the composition of the fuel mixture on the basis of the received characteristic value of the fuel mixture. The identified composition of the fuel mixture or the information regarding the composition may be memorized in a storage 23 of the composition identification apparatus 2, or may be transmitted from the composition identification apparatus 2 to another computer apparatus 7 by communication. In another computer apparatus 7, the received composition of the fuel mixture or information regarding the composition can be memorized in a storage. In addition, the composition of the fuel mixture identified by the composition identification apparatus 2 provided outside the moving object 5 or information regarding the composition may be transmitted to the moving object 5 by wireless communication. The composition of the fuel mixture or information regarding the composition may be received by a communication interface provided in the moving object 5, and the received information may be memorized in a storage provided in the moving object 5.

Regardless of whether the composition identification apparatus 2 is provided inside or outside the moving object 5, the composition of a fuel mixture identified by the composition identification apparatus 2 or the information regarding the composition may be displayed on a display unit provided in the moving object 5 so that a person riding on the moving object 5 can grasp the composition of the fuel mixture or the information regarding the composition.

Fig. 2 is a block diagram illustrating a configuration of a composition identification apparatus according to an embodiment of the present invention. The composition identification apparatus 2 includes a control unit 21, a main memory 22, a storage 23, and a communication interface 24, which are connected by a bus.

The control unit 21 includes a CPU and a ROM. The control unit 21 executes a program stored in the storage 23 and controls the composition identification apparatus 2. As the main memory 22, for example, a RAM is used. The main memory 22 is a work area of the control unit 21. The storage 23 is a memory area for saving programs and data. The control unit 21 performs arithmetic processing on the basis of programs and data read from the main memory 22.

The communication interface 24 can be connected to the communication network 6 wirelessly or by wire. Data received via the communication interface 24 is loaded into the main memory 22, and arithmetic processing is performed by the control unit 21.

Another computer apparatus 7 may have a configuration similar to that of the composition identification apparatus 2. More specifically, another computer apparatus 7 includes a control unit, a main memory, a storage, and a communication interface, which are connected by a bus. The application and purpose of the composition identification apparatus 2 of another computer apparatus 7 are not particularly limited. In addition, another computer apparatus 7 may be provided inside the moving object 5 or may be provided outside the moving object 5. Another computer apparatus 7 may be, for example, a server apparatus that memorizes the composition of the fuel mixture identified by the plurality of composition identification apparatuses 2 or information regarding the composition, or may be a computer apparatus provided inside the moving object 5.

Next, composition identification processing will be described. The composition identification apparatus 2 can be mounted on, for example, a diesel vehicle. A diesel vehicle includes a fuel tank for supplying fuel to an engine, and the fuel tank stores the fuel.

Here, a case where a mixture of three types of fuels including fossil fuel (for example, equivalent to liquid A), hydrogenated vegetable oil (for example, equivalent to liquid B), and bio-derived fuel (for example, equivalent to liquid C) is stored as the fuel will be described. However, the fuel mixture may be a combination of different types of fuels from these three types of fuels. Examples of the bio-derived fuel include fuel oil containing a fatty acid methyl ester.

The fuel tank includes measurers capable of measuring a characteristic value of a fuel mixture. Here, a case where a dielectric constant sensor is provided as a measurer for measuring the first characteristic value, and a thermal conductivity sensor is provided as a measurer for measuring the second characteristic value will be described. The dielectric constant sensor can measure a dielectric constant of the fuel mixture in the fuel tank or a parameter for calculating the dielectric constant, and the thermal conductivity sensor can measure a thermal conductivity of the fuel mixture in the fuel tank or a parameter for calculating the thermal conductivity. The fuel tank may further include a temperature sensor capable of measuring the temperature of the fuel mixture.

Here, the dielectric constant or the parameter for calculating the dielectric constant is adopted as the first characteristic value, and the thermal conductivity or the parameter for calculating the thermal conductivity is adopted as the second characteristic value, but the combination of the first characteristic value and the second characteristic value is not limited thereto. For example, in addition to the dielectric constant and the thermal conductivity, a characteristic value that linearly (or substantially linearly) changes according to the composition when different types of fuels are mixed may be used as the first characteristic value and/or the second characteristic value. That is, the characteristic value that changes in proportion to the content of each fuel in the fuel mixture can be adopted as the first characteristic value and/or the second characteristic value. Here, the "substantially linear change" refers to a case where the characteristic value tends to increase or decrease as the content of a specific component increases, or the characteristic value tends to increase or decrease as the content of a specific component decreases, and a change different from a complete linear change is made to such an extent that an appropriate content can be identified by correction. Examples of the characteristic value used in addition to the dielectric constant and the thermal conductivity include a kinematic viscosity, an acid value, a density, and the like. In addition, as the first characteristic value and the second characteristic value, it is also possible to adopt a characteristic value which is not the characteristic value itself such as the dielectric constant, the thermal conductivity, and the like, but has a correlation with a characteristic value such as the dielectric constant, the thermal conductivity, and the like, for example, capacitance or a voltage output when the dielectric constant or the thermal conductivity is measured by a sensor.

Fig. 3 is a flowchart illustrating composition identification processing according to an embodiment of the present invention. The order of each processes constituting the flowchart described below may be a random order as long as there is no contradiction or inconsistency in the processing contents. The composition identification processing is executed by the composition identification apparatus 2.

First, information regarding the dielectric constant P of the fuel mixture in the fuel tank measured by the dielectric constant sensor is received by the composition identification apparatus 2 (step S1). The information regarding the dielectric constant P may be the dielectric constant P itself or a parameter regarding the dielectric constant P. The parameter regarding the dielectric constant P means that the dielectric constant P can be calculated using the parameter. The parameter regarding the dielectric constant P is preferably a parameter having a proportional relationship or an inverse proportional relationship with the dielectric constant P. Examples of the parameter regarding the dielectric constant P include "capacitance" in a case where a capacitor is manufactured by inserting a fuel mixture to be measured between two parallel sheet-shaped electrodes, and the capacitance of the manufactured capacitor is measured. The "capacitance" is a parameter having a proportional relationship with the dielectric constant P.

As a method for measuring the capacitance, a known method can be used. For example, the capacitance can be calculated by disposing electrodes such that a fuel mixture to be measured is between two flat metal electrodes, applying a voltage between the electrodes, and measuring the impedance of a capacitor formed by the two electrodes.

Next, the composition identification apparatus 2 receives information regarding a thermal conductivity Q of the fuel mixture in the fuel tank measured by the thermal conductivity sensor (step S2). The information regarding the thermal conductivity Q may be the thermal conductivity Q itself or a parameter regarding the thermal conductivity Q. The parameter regarding the thermal conductivity Q means that the thermal conductivity Q can be calculated using the parameter. The parameter regarding the thermal conductivity Q is preferably a parameter having a proportional relationship or an inverse proportional relationship with the thermal conductivity Q.

Differential scanning calorimetry (DSC) is a method in which a blank and a measurement sample are heated under the same conditions, and a specific heat capacity is measured from a temperature difference between the blank and the measurement sample, which is generated when the blank and the measurement sample absorb and generate heat. The blank is, for example, an empty container identical to the container enclosing the measurement sample. By measuring the specific heat capacity, the thermal conductivity can be identified. The temperature difference between the blank and the measurement sample causes a difference in electromotive force between two thermocouples in the differential scanning calorimetry. This electromotive force difference "dVx" can be used as a parameter regarding the thermal conductivity Q. "dVx" is a parameter having an inverse proportional relationship with the thermal conductivity Q.

In addition, as a method of measuring "dVx", the following method can also be used. Fig. 10 is a diagram illustrating a thermal conductivity sensor according to an embodiment of the present invention. The shape of a thermal conductivity sensor 30 is not particularly limited, and can be, for example, a sheet shape. Fig. 10 is a side view of the sheet-shaped thermal conductivity sensor. The thermal conductivity sensor 30 includes temperature sensors 31a and 31b, and a heater 32. As the temperature sensors 31a and 31b, known sensors can be used. The temperature sensors 31a and 31b are made of the same material. Furthermore, it is preferable that the temperature sensors 31a and 31b have a sheet shape. It is preferable that the temperature sensors 31a and 31b have the same size and shape. For the temperature sensor 31a, the heater 32 is brought into contact with the temperature sensor 31a, or the heater 32 is disposed in the vicinity of the temperature sensor 31a. For the other temperature sensor 31b, the heater 32 is not brought into contact with the temperature sensor 31b, and the heater 32 is not disposed in the vicinity of the temperature sensor 31b. The temperature sensor 31b and the periphery thereof are preferably not affected by the temperature increase of the heater 32. When the temperature of the heater 32 is increased in this manner, the temperature around the temperature sensor 31a and the temperature around the temperature sensor 31b are set to be different from each other. The two temperature sensors 31 and the heater 32 are covered with an insulator 33, thereby forming the thermal conductivity sensor 30. The thermal conductivity sensor 30 is brought into contact with a liquid such as a fuel mixture 34 to be measured, for example, by immersing the sensor in the liquid. The rate of increase in the temperature of the heater 32 is preferably constant in any measurement so that dVx becomes the same value when the same fuel mixture 34 is measured under the environment of the same air pressure and temperature. When the temperature of the heater 32 increases, the output voltage of the temperature sensor 31a changes, but the output voltage of the temperature sensor 31b does not change. The difference between the output voltages is dVx.

Furthermore, information regarding the temperature of the fuel mixture in the fuel tank measured by the temperature sensor is received by the composition identification apparatus 2 (step S3).

The three types of fuels contained in the fuel mixture have dielectric constants and thermal conductivities unique to the respective fuels, and these dielectric constants and thermal conductivities change depending on the state of the fuel mixture or the surrounding environment of the fuel mixture (hereinafter, referred to as a state of a fuel mixture or the like). For example, the dielectric constant and thermal conductivity of each fuel change depending on the temperature of the fuel. Therefore, the information regarding dielectric constants $P_A$ to Pc and the information regarding thermal conductivities $Q_A$ to Qc used for identifying the composition of the fuel mixture in step S5 are identified on the basis of the temperature of the fuel mixture in the fuel tank received in step S3 (step S4). Here, the correction of the value of the capacitance on the basis of the temperature of the fuel mixture will be described. However, if the characteristic value changes according to a parameter indicating the state of the fuel mixture or the surrounding environment other than the temperature of the fuel mixture, the characteristic value can be corrected according to the parameter.

Fig. 4 is a diagram illustrating a relationship between the dielectric constant and the temperature of three types of fuels. The horizontal axis corresponds to the voltage output from the temperature sensor, and the higher the voltage, the higher the temperature. The vertical axis corresponds to the capacitance value output by the dielectric constant sensor, and the higher the capacitance value, the higher the dielectric constant. In Fig. 4, it can be seen that the dielectric constant of the bio-derived fuel (BDF) (registered trademark) decreases as it changes from 10°C to 50°C. In addition, it can be seen that the dielectric constant of the hydrogenated vegetable oil (HVO) and the dielectric constant of the fossil fuel hardly change even when changing from 10°C to 50°C.

For example, an approximate formula of a correlation between a dielectric constant (or a parameter regarding the dielectric constant) and a temperature is set for each of the three types of fuels, and on the basis of the temperature of the fuel mixture in the fuel tank received in step S3, unique dielectric constants $P_A$ to Pc of each of the three types of fuels at the temperature (or a parameter regarding the unique dielectric constant of each of the three types of fuels) can be calculated. As the approximate formula, known approximate formulas such as exponential approximation, linear approximation, logarithmic approximation, and polynomial approximation can be used. In the case of polynomial approximation, a linear formula or a quadratic formula may be used, and the order is not particularly limited. In addition, unlike the method of calculating the dielectric constant using the approximate formula, for example, a data table memorizing a correspondence relationship between the temperature and the dielectric constant (or a parameter regarding the dielectric constant) is set every 0.1°C or every 0.01°C for each of the three types of fuels, and unique dielectric constants $P_A$ to Pc (or a parameter regarding the unique dielectric constant of each of the three types of fuels) corresponding to the temperature of the fuel mixture in the fuel tank can be identified with reference to the data table.

Fig. 4 illustrates the relationship between the dielectric constant and the temperature in the three types of fuels, but the thermal conductivity also has a correlation with the temperature. Similarly, regarding the thermal conductivity, an approximate formula of the correlation between the thermal conductivity (or the parameter regarding the thermal conductivity) and the temperature is set for each of the three types of fuels, and the unique thermal conductivities $Q_A$ to Qc (or the parameter regarding the unique thermal conductivity of each of the three types of fuels) of each of the three types of fuels at the temperature can be calculated on the basis of the temperature of the fuel mixture in the fuel tank received in step S3. Similarly to the dielectric constant, a data table memorizing a correspondence relationship between the temperature and the thermal conductivity (or a parameter regarding the thermal conductivity) is set every 0.1°C or every 0.01°C, and unique thermal conductivities $Q_A$ to Qc (or a parameter regarding the unique thermal conductivity of each of the three types of fuels) corresponding to the temperature of the fuel mixture in the fuel tank can be identified with reference to the data table.

Next, the composition of the fuel mixture is identified on the basis of the received the information regarding the dielectric constant P and the thermal conductivity Q of the fuel mixture and the information regarding the dielectric constants $P_A$ to Pc and the thermal conductivities $Q_A$ to Qc unique to the three types of fuels identified in step S4 (step S5).

Fig. 5 is a diagram illustrating a relationship between the dielectric constant and the thermal conductivity of three types of fuels. The vertical axis represents a parameter "dVx" regarding the thermal conductivity. The horizontal axis represents a parameter "capacitance" regarding the dielectric constant. The dielectric constant and thermal conductivity of fatty acid methyl ester derived from soybean (SME), hydrogenated vegetable oil (HVO), and light oil (fossil fuel) have different values. In Fig. 5, there is provided a triangle having three points as vertices obtained by plotting, for each of these fuels, the parameter "capacitance" regarding the dielectric constant and the parameter "dVx" regarding the thermal conductivity. When the parameter "capacitance" regarding the dielectric constant and the parameter "dVx" regarding the thermal conductivity of the fuel mixture obtained by mixing these three types of fuels are plotted in Fig. 5, the parameters can be plotted inside the triangle.

For example, the dielectric constant and thermal conductivity of a fuel mixture with a volume ratio of 50 : 50 of light oil to HVO correspond to the dielectric constant and thermal conductivity at point AB that is intermediate between point A plotted for light oil and point B plotted for HVO. A case where "capacitance" and "dVx" of a fuel mixture of light oil and HVO at a volume ratio of 50 : 50 are plotted also corresponds to the "capacitance" and "dVx" of point A'B' intermediate between point A' plotted for light oil and point B' plotted for HVO. The dielectric constant and thermal conductivity of a fuel mixture of BDF (registered trademark) (SME 100) and light oil at a volume ratio of 20 : 80 correspond to the dielectric constant and thermal conductivity of point CA obtained by dividing the distance between point C and point A such that the distance from point C is 20% and the distance from point A is 80% on a line segment between point C plotted for BDF (registered trademark) and point A plotted for light oil. A case where "capacitance" and "dVx" of a fuel mixture of BDF (registered trademark) (SME 100) and light oil at a volume ratio of 20 : 80 are plotted also corresponds to "capacitance" and "dVx" of point C'A' intermediate between point C' plotted for BDF (registered trademark) and point A' plotted for light oil. Furthermore, the dielectric constant and thermal conductivity of a fuel mixture of light oil, HVO, and BDF (registered trademark) at a volume ratio of 65 : 28 : 7 correspond to the dielectric constant and thermal conductivity of point ABC moved to the inside of the triangle toward point C from a point at which division is allowed such that the distance between points A and B satisfies the distance from point A : the distance from point B = 65 : 28 on a line segment between point A plotted for light oil and point B plotted for HVO. A case where "capacitance" and "dVx" of a fuel mixture of light oil, HVO, and BDF (registered trademark) at a volume ratio of 65 : 28 : 7 are plotted also corresponds to "capacitance" and "dVx" of point A'B'C' moved to the inside of the triangle toward point C' from a point at which division is allowed such that the distance between point A' and point B' on the line segment between point A' plotted for light oil and point B' plotted for HVO satisfies the distance from point A' : the distance from point B' = 65 : 28.

As described above, by using the characteristic value that changes in proportion to the content of each fuel in the fuel mixture (including the characteristic value that changes in substantially proportion to the content of each fuel in the fuel mixture), the composition of the fuel mixture can be identified by the following formula. Here, the dielectric constant of the fuel mixture is defined as $P(Fm^{-1})$, and the thermal conductivity of the fuel mixture is defined as $Q(W/(m \cdot K))$. In addition, the dielectric constant of fossil fuel is defined as $P_A(Fm^{-1})$, the thermal conductivity of fossil fuel is defined as $Q_A(W/(m \cdot K))$, the dielectric constant of hydrogenated vegetable oil is defined as $P_B(Fm^{-1})$, the thermal conductivity of hydrogenated vegetable oil is defined as $Q_B(W/(m \cdot K))$, the dielectric constant of bio-derived fuel is defined as $Pc(Fm^{-1})$, and the thermal conductivity of bio-derived fuel is defined as $Qc(W/(m \cdot K))$. In step S5, as the composition of the fuel mixture, a volume fraction X (vol%) of fossil fuel, a volume fraction Y (vol%) of hydrogenated vegetable oil, and a volume fraction Z (vol%) of bio-derived fuel can be identified by the following formulas (a) to (c).

[Mathematical formula 2]

$$X = 100 - Y - Z \quad \cdots (a)$$

$$Y = \frac{(P - P_A)(Q_C - Q_A) - (P_C - P_A)(Q - Q_A)}{(P_B - P_A)(Q_C - Q_A) - (P_C - P_A)(Q_B - Q_A)} \times 100 \quad \cdots (b)$$

$$Z = \frac{-(P - P_A)(Q_B - Q_A) + (P_B - P_A)(Q - Q_A)}{(P_B - P_A)(Q_C - Q_A) - (P_C - P_A)(Q_B - Q_A)} \times 100 \quad \cdots (c)$$

The values of the dielectric constant $P_A(Fm^{-1})$ and the thermal conductivity $Q_A(W/(m \cdot K))$ of fossil fuel, the dielectric constant $P_B(Fm^{-1})$ and the thermal conductivity $Q_B(W/(m \cdot K))$ of hydrogenated vegetable oil, and the dielectric constant $Pc(Fm^{-1})$ and the thermal conductivity $Qc(W/(m \cdot K))$ of bio-derived fuel are memorized in advance in the main memory or the storage of the composition identification apparatus 2, and are used when identifying the composition of the fuel mixture in step S5. All of the volume fraction X (vol%) of fossil fuel, the volume fraction Y (vol%) of hydrogenated vegetable oil, and the volume fraction Z (vol%) of bio-derived fuel may be identified by the formulas (a) to (c), and any one or two of the volume fraction X (vol%) of fossil fuel, the volume fraction Y (vol%) of hydrogenated vegetable oil, and the volume fraction Z (vol%) of bio-derived fuel may be identified by any one or two of the formulas (a) to (c). When any one or two of the volume fraction X (vol%) of fossil fuel, the volume fraction Y (vol%) of hydrogenated vegetable oil, and the volume fraction Z (vol%) of bio-derived fuel are identified by any one or two of the formulas (a) to (c), the volume fraction of the remaining fuel may be identified using other methods.

In the above formulas (a) to (c), the volume fractions of the three types of fuels can be calculated as the composition of the fuel mixture, but the mass fractions of the three types of fuels can also be calculated from the specific gravities of the three types of fuels on the basis of the calculated volume fractions. The mass fraction of the three fuels can also be memorized as the composition of the fuel mixture.

In step S5, the composition of the fuel mixture can be identified by substituting the dielectric constant P and the thermal conductivity Q of the fuel mixture, and the dielectric constants $P_A$ to $P_C$ and the thermal conductivities $Q_A$ to Qc unique to the three types of fuels into the above formulas (a) to (c). However, the composition of the fuel mixture can be identified by the above formulas (a) to (c) by using the parameter P regarding the dielectric constant of the fuel mixture and the parameter Q regarding the thermal conductivity instead of the dielectric constant P and the thermal conductivity Q of the fuel mixture, and substituting the parameters $P_A$ to Pc regarding the dielectric constant and the parameters $Q_A$ to Qc regarding the thermal conductivity unique to the three types of fuels instead of the dielectric constant $P_A$ to $P_C$ and the thermal conductivity $Q_A$ to Qc unique to the three types of fuels. For example, a detection value (for example, a capacitance value detected by the sensor) detected by a dielectric constant sensor is used as the parameter P regarding the dielectric constant of the fuel mixture, and a detection value (for example, dVx detected by the sensor) detected by a thermal conductivity sensor is used as the parameter Q regarding the thermal conductivity of the fuel mixture. Similarly, the composition of the fuel mixture can be identified using the parameters $P_A$ to Pc regarding the dielectric constants unique to the three types of fuels, that is, the detection values (for example, values of capacitances detected by the sensor) of the dielectric constant sensor corresponding to the dielectric constants, and the parameters regarding the thermal conductivities unique to the three types of fuels, that is, the detection values (for example, dVx detected by the sensor) of the thermal conductivity sensor corresponding to the thermal conductivities.

Next, the composition of the fuel mixture identified in step S5 is memorized in the storage 23 of the composition identification apparatus 2 or transmitted to another computer apparatus 7 (step S6). Either the memorization of the composition of the fuel mixture in the storage 23 or the transmission of the composition of the fuel mixture to another computer apparatus 7 may be executed, or both of them may be executed. When the composition of the fuel mixture is received by another computer apparatus 7, the received composition of the fuel mixture is memorized in the storage 23 of another computer apparatus 7. The composition identification processing ends by the processes of steps S1 to S6.

When the composition of the fuel mixture is memorized in the storage 23 of the composition identification apparatus 2 or the storage of another computer apparatus 7, the identified composition is memorized in association with, for example, a time when a characteristic value such as a dielectric constant or a thermal conductivity is measured, a time when the characteristic value is received in step S1 and/or step S2, or a time when the composition of the fuel mixture in step S5 is identified.

In addition, the composition identification processing in steps S1 to S6 is preferably executed periodically at predetermined time intervals. The cycle at which the composition identification processing is executed can be appropriately set. For example, the composition identification processing may be executed every 1 minute, every 1 hour, or every 24 hours. The composition identification processing in steps S1 to S6 may be executed every time a predetermined condition is satisfied, for example, every time fuel is supplied to the fuel tank. As described above, the identified composition is memorized in association with time, and then the composition identification processing in steps S1 to S6 is repeatedly executed a plurality of times, whereby the composition history can be memorized.

In the above step S5, the case where the composition of the fuel mixture is identified using the formulas (a) to (c) has been described. Hereinafter, a method of identifying the composition of the fuel mixture by another method in step S5 will be described.

As illustrated in Fig. 7, in the graph in which the parameter regarding the thermal conductivity is plotted on the vertical axis and the parameter regarding the dielectric constant is plotted on the horizontal axis, the point where the mixed oil of light oil and BDF (registered trademark) is plotted is not on a straight line connecting the point where light oil is plotted and the point where BDF (registered trademark) is plotted. Similarly, the point where the mixed oil of HVO and BDF (registered trademark) is plotted is not on a straight line connecting the point where HVO is plotted and the point where BDF (registered trademark) is plotted. This means that the actual thermal conductivity when two different fuel mixtures are mixed is lower than the value that can be calculated from the thermal conductivity and mixing ratio of the respective fuel mixture. Since dVx on the vertical axis in Fig. 5 has a smaller value as the thermal conductivity increases and a larger value as the thermal conductivity decreases, dVx when two different fuel mixtures are mixed is higher than a value that can be calculated from the thermal conductivity and the mixing ratio of the respective fuel mixture. Therefore, correction is required in identifying the composition of the fuel mixture.

In step S5, a method of obtaining the composition of the fuel mixture using curve approximation will be described. The method of obtaining using curve approximation is performed by the following procedure. In obtaining the composition of the fuel mixture using curve approximation, reference is made to Fig. 8. In (1) to (9) below, it is preferable to use the measured values at the temperature of the fuel mixture measured in step S3 as dVx and capacitance of light oil, HVO, and BDF (registered trademark). In addition, it is preferable to use measured values at the temperature of the fuel mixture measured in step S3 also for dVx and capacitance of a plurality of mixtures in which the proportion of HVO is 0 vol% and the compositions of light oil and BDF (registered trademark) are different from each other. It is preferable to use measured values at the temperature of the fuel mixture measured in step S3 also for dVx and capacitance of a plurality of mixtures in which the proportion of light oil is 0 vol% and the compositions of HVO and BDF (registered trademark) are different from each other.

(1) A formula representing a straight line connecting a point where dVx and capacitance of light oil are plotted and a point where dVx and capacitance of HVO are plotted is obtained.
(2) dVx and capacitance of the fuel mixture are measured, and a formula representing a straight line connecting a point plotted on the basis of the measured result and a point where dVx and capacitance of BDF (registered trademark) are plotted is obtained.
(3) An intersection point 1 between the straight line obtained in the (1) and the straight line obtained in the (2) is obtained.
(4) The proportion of BDF (registered trademark) in the fuel mixture is obtained by the following formula (d).
[Mathematical formula 3]

$$\text{Proportion of fuel mixture in BDF (registered trademark)} = \text{(capacitance of fuel mixture} - \text{capacitance at intersection point 1)} / \text{(capacitance of BDF} - \text{capacitance at intersection point 1)} \cdots \text{(d)}$$

(5) The straight line obtained in the (1) is translated so as to include a point plotted on the basis of the measurement result.
(6) An intersection point 2 between a fitting curve and the straight line translated in the (5) is obtained, the fitting curve being obtained from a point where dVx and capacitance of a plurality of mixtures in which the proportion of HVO is 0 vol% and the compositions of light oil and BDF (registered trademark) are different from each other are plotted.
(7) An intersection point 3 between a fitting curve and the straight line translated in the (5) is obtained, the fitting curve being obtained from a point where dVx and capacitance of a plurality of mixtures in which the proportion of light oil is 0 vol% and the compositions of HVO and BDF (registered trademark) are different from each other are plotted.
(8) The proportion of the HVO is obtained by the following formula (e). Here, the proportion of the HVO obtained by the formula (e) is a proportion of the HVO to the residue obtained by subtracting the proportion of BDF (registered trademark) in the fuel mixture from 100%. The proportion of the HVO in the fuel mixture is obtained from the proportion of the HVO obtained by the formula (e).
[Mathematical formula 4]

Proportion of HVO = (dVx at intersection point 2 - dVx of fuel mixture) / (dVx at intersection point 2 - dVx at intersection point 3) × (100% - proportion of fuel mixture in BDF (registered trademark)) ···(e)

(9) The proportion of BDF (registered trademark) in the fuel mixture and the proportion of HVO in the fuel mixture are subtracted from 100% to obtain the proportion of light oil in the fuel mixture.

The fitting curve refers to a curve obtained by fitting a curve so as to apply to a plurality of pieces of data obtained in an experiment. Examples of the curve fitting method include a method of estimating an optimum function by a least squares method. Hereinafter, the same applies to the fitting curve.

The proportion of BDF (registered trademark) in the fuel mixture theoretically obtained from dVx and capacitance of each of light oil, HVO, and BDF (registered trademark) and dVx and capacitance of the fuel mixture is less deviated from the actual BDF (registered trademark) as compared with the case of HVO and light oil. Therefore, in (4), the proportion of BDF (registered trademark) in the fuel mixture can be calculated, and the proportion of HVO and light oil in the fuel mixture can be obtained using the fitting curve and the calculated proportion of BDF (registered trademark) in the fuel mixture.

Note that, although dVx and capacitance are used as the characteristic values here, the present method can be used even when other characteristic values are used.

Next, in step S5, a method for obtaining the composition of the fuel mixture using curve approximation different from the above will be described. When this method is used, step S4 is omitted.

(1) A plurality of mixtures in which the proportion of HVO is 0 vol% and the mixing ratio of light oil and BDF (registered trademark) is changed are prepared, and dVx and capacitance are measured by changing the temperature. Similarly, a plurality of mixtures in which the proportion of light oil is 0 vol% and the mixing ratio of HVO and BDF (registered trademark) is changed are prepared, and dVx and capacitance are measured by changing the temperature.
(2) A fitting curve representing a relationship between dVx and capacitance of a mixture of light oil and BDF (registered trademark) and a fitting curve representing a relationship between dVx and capacitance of a mixture of HVO and BDF (registered trademark) are obtained for each temperature.
(3) From a plurality of fitting curves obtained for each temperature, a temperature-dependent formula representing a relationship between dVx and capacitance of a mixture of light oil and BDF (registered trademark) and a temperature-dependent formula representing a relationship between dVx and capacitance of a mixture of HVO and BDF (registered trademark) are obtained.
The (1) to (3) are executed in advance, and these temperature dependent formulas are memorized in the storage.
In step S5, the following processes (4) to (6) are executed.
(4) From the temperature measured in step S3, temperature correction is performed for the above two temperature dependent formulas.
(5) The proportion of the fuel mixture of BDF (registered trademark) is calculated on the basis of the following equation (c).
[Mathematical formula 5]

$$Z = \frac{-(P-P_A)(Q_B-Q_A)+(P_B-P_A)(Q-Q_A)}{(P_B-P_A)(Q_C-Q_A)-(P_C-P_A)(Q_B-Q_A)} \times 100 \quad \cdots (c)$$

(6) The proportion of light oil in the fuel mixture is calculated using BDF (registered trademark) calculated in the (5) and the formula obtained by correcting the temperature of light oil-BDF (registered trademark) in the (4). In addition, the proportion of the HVO in the fuel mixture is calculated using BDF (registered trademark) calculated in (5) and the formula obtained by correcting the temperature of HVO-BDF (registered trademark) in the (4).

As described above, the proportion of BDF (registered trademark) in the fuel mixture theoretically obtained from dVx and capacitance of each of light oil, HVO, and BDF (registered trademark) and dVx and capacitance of the fuel mixture is less deviated from the actual BDF (registered trademark) as compared with the case of HVO and light oil. Therefore, in (5), the proportion of BDF (registered trademark) in the fuel mixture can be calculated by the formula (c), and the proportion of HVO and light oil in the fuel mixture can be obtained using the fitting curve and the calculated proportion of BDF (registered trademark) in the fuel mixture.

Next, a method of correcting the composition of the fuel mixture on the basis of the fitting curve of the deviation amount in step S5 will be described. Fig. 9 is a graph in which, for a fuel mixture including two components of HVO and BDF (registered trademark), the deviation amount of the actual proportion of HVO in the fuel mixture from the proportion of HVO calculated by the formula (b) on the basis of the measured dVx and capacitance is plotted on the vertical axis, and the proportion of BDF (registered trademark) in the fuel mixture is plotted on the horizontal axis. Furthermore, for a fuel mixture including two components of light oil and BDF (registered trademark), the deviation amount of the actual proportion of HVO in the fuel mixture from the proportion of light oil calculated by formulas (a) to (c) on the basis of the measured dVx and capacitance is plotted on the vertical axis, and the proportion of BDF (registered trademark) in the fuel mixture is plotted on the horizontal axis. The deviation amount is, for example, a difference obtained by subtracting the proportion of the HVO calculated by the formula (b) from the actual proportion of the HVO in the fuel mixture. Similarly, the deviation amount is, for example, a difference obtained by subtracting the proportion of light oil calculated by the formulas (a) to (c) from the actual proportion of light oil in the fuel mixture.

As illustrated in Fig. 9, a fitting curve is obtained from points plotted on the basis of the deviation amount and the proportion of BDF (registered trademark) in the fuel mixture for a fuel mixture including a plurality of two components.

For example, it is possible to calculate the deviation amount for fuel mixtures having different proportions of BDF (registered trademark) for each temperature, perform curve fitting using the proportion of BDF (registered trademark) as an explanatory variable and the deviation amount as an objective variable for each temperature, obtain a fitting curve for each temperature, and obtain a fitting formula representing the fitting curve for each temperature.

It is also possible to calculate the deviation amount for fuel mixtures having different proportions of BDF (registered trademark) at a plurality of different temperatures, perform curve fitting using the proportion of BDF (registered trademark) as an explanatory variable and the deviation amount as an objective variable, and obtain a fitting formula representing one fitting curve applicable even at a plurality of different temperatures.

In addition, unlike Fig. 9, for a fuel mixture including two components of HVO and BDF (registered trademark), it is also possible to obtain a fitting formula representing a relationship between a deviation amount of an actual proportion of HVO in the fuel mixture from a proportion of HVO calculated by the formula (b) on the basis of the measured dVx and capacitance and a proportion of BDF (registered trademark) in the fuel mixture. Similarly, for a fuel mixture including two components of light oil and BDF (registered trademark), it is also possible to obtain a fitting formula representing the relationship between the deviation amount of the actual proportion of light oil in the fuel mixture from the proportion of light oil calculated by the formulas (a) to (c) on the basis of the measured dVx and capacitance and the proportion of BDF (registered trademark) in the fuel mixture. Also in this case, it is possible to calculate the deviation amount for fuel mixtures having different proportions of BDF (registered trademark) for each temperature, perform curve fitting using the proportion of BDF (registered trademark) as an explanatory variable and the deviation amount as an objective variable for each temperature, obtain a fitting curve for each temperature, and obtain a fitting formula representing the fitting curve for each temperature. It is also possible to calculate the deviation amount for fuel mixtures having different proportions of BDF (registered trademark) at a plurality of different temperatures, perform curve fitting using the proportion of BDF (registered trademark) as an explanatory variable and the deviation amount as an objective variable, and obtain one fitting curve applicable even at a plurality of different temperatures. A fitting formula representing the fitting curve can also be obtained.

For a fuel mixture whose composition has not been identified, the deviation amount can be obtained by identifying the proportion of BDF (registered trademark) by the formula (c) and then inputting the proportion of BDF (registered trademark) as an explanatory variable to a fitting formula. Alternatively, it is also possible to prepare in advance a table defining a correspondence relationship between a proportion of different BDF (registered trademark) and a deviation amount using this fitting formula and identify the deviation amount according to the proportion of BDF (registered trademark). Such fitting formula or table is memorized in the storage in advance.

In step S5, first, the proportion of BDF (registered trademark) in the fuel mixture is calculated using the formula (c) on the basis of the measured values of dVx and capacitance of the fuel mixture. Then, on the basis of the calculated proportion of BDF (registered trademark) in the fuel mixture, the deviation amount of the proportion of HVO or light oil is identified on the basis of the fitting formula or table memorized in the storage. Then, the proportion of HVO or the proportion of light oil can be calculated by adding the deviation amount to the proportion of HVO or the proportion of light oil calculated by the formulas (a) to (c) on the basis of dVx and capacitance measured for the fuel mixture. Note that the proportion of light oil can also be calculated from the proportion of HVO and the proportion of BDF (registered trademark) after calculating the proportion of HVO.

As described above, in the present invention, the composition of the fuel mixture to be measured can be identified on the basis of the correspondence relationship (for example, the fitting formula or the table) between the volume fraction of the liquid in the fuel mixture calculated by the predetermined calculation formula (in the case of three components, for example, the formula (a), the formula (b), and/or the formula (c)) and the deviation amount of the volume fraction of each liquid in the actual fuel mixture, and further on the basis of the characteristic value of the fuel mixture to be measured.

Here, the deviation amount is a difference obtained by subtracting a theoretically calculated proportion of a certain component from an actual proportion of the certain component in the fuel mixture. However, a more accurate proportion of the component can be identified from the theoretically calculated proportion of the component from the characteristic value of the fuel mixture to be measured on the basis of a ratio of the actual proportion of the component in the fuel mixture to the theoretically calculated proportion of the certain component in the fuel mixture. In this case, by multiplying the proportion of the components theoretically calculated from the characteristic value of the fuel mixture to be measured by the ratio, a more accurate proportion of the components can be identified.

Note that, in the embodiment described above, identification of the composition of the fuel mixture is mainly described. However, the composition identification apparatus and the composition identification system of the present invention can be used not only for identifying the composition of the fuel mixture but also for identifying the composition of a mixture obtained by mixing a plurality of different liquids used for applications other than fuel. The liquid contained in the mixture is not particularly limited, and water, an organic solvent, and the like are candidates. The organic solvent may be either flammable or non-flammable, and may be either volatile or nonvolatile.

Fig. 6 is a diagram illustrating a composition identification apparatus according to an embodiment of the present invention. Fig. 6A is a perspective view of the composition identification apparatus, and Fig. 6B is a side view of the composition identification apparatus. The composition identification apparatus 2 can be attached to a fuel tank of a vehicle such as an automobile by a tank attaching unit 25. The composition identification apparatus 2 includes a sensor unit 26 and a circuit unit 27. The sensor unit 26 includes a dielectric constant sensor, a thermal conductivity sensor, and a temperature sensor. When the composition identification apparatus is attached to the fuel tank, the fuel mixture in the fuel tank is supplied to the sensor unit 26, and the dielectric constant, the thermal conductivity, and the temperature of the fuel mixture can be measured. In a case where a value other than the dielectric constant and the thermal conductivity is adopted as the first characteristic value and the second characteristic value, the sensor unit 26 may include a sensor capable of measuring these characteristic values.

The circuit unit 27 includes the control unit 21, the main memory 22, the storage 23, and the communication interface 24. The information regarding the dielectric constant, the thermal conductivity, and the temperature measured by the sensor unit 26 is received by the communication interface 24. The control unit 21 executes the composition identification processing in steps S1 to S6 on the basis of the received information to identify the composition of the fuel mixture, and memorizes the obtained composition of the fuel mixture in the storage 23. Instead of identifying the composition of the fuel mixture by the apparatus attached to the fuel tank, the information regarding the dielectric constant, the thermal conductivity, and the temperature measured by the dielectric constant sensor, the thermal conductivity sensor, and the temperature sensor in the apparatus attached to the fuel tank may be transmitted to another computer apparatus 7, and the composition of the fuel mixture may be identified by another computer apparatus 7.

According to the present invention, the composition of the mixture can be identified on the basis of the first characteristic value and the second characteristic value of the mixture, the first characteristic value and the second characteristic value unique to the liquid A, the first characteristic value and the second characteristic value unique to the liquid B, and the first characteristic value and the second characteristic value unique to the liquid C. Furthermore, according to the present invention, the composition of the mixture in the fuel tank can be identified on the basis of the first characteristic value and the second characteristic value of the mixture stored in the fuel tank of the heat engine.

According to the present invention, the first characteristic value and/or the second characteristic value unique to the liquid A, the first characteristic value and/or the second characteristic value unique to the liquid B, and/or the first characteristic value and/or the second characteristic value unique to the liquid C can be identified according to the temperature of the mixture.

According to the present invention, the composition of the mixture can be identified on the basis of the dielectric constant or the parameter regarding the dielectric constant and the thermal conductivity or the parameter regarding the thermal conductivity of the mixture.

According to the present invention, since the liquid A is fossil fuel, the liquid B is hydrogenated vegetable oil, and the liquid C is bio-derived fuel, it is possible to identify the composition of a mixture including fossil fuel, hydrogenated vegetable oil, and bio-derived fuel.

According to the present invention, changes in the composition of the mixture can be recorded as a history in order to memorize the composition of the identified mixture.

According to the present invention, since the identified composition of the mixture is transmitted to another computer apparatus, changes in the composition of the mixture can be recorded as a history in another computer apparatus.

According to the present invention, the composition of the fuel mixture can be identified on the basis of one or a plurality of characteristic values of the fuel mixture including different types of fuels and one or a plurality of characteristic values unique to each of the plurality of fuels included in the fuel mixture.

According to the present invention, since one or a plurality of characteristic values unique to each of the plurality of fuels are values identified according to the state of the fuel mixture or the surrounding environment of the fuel mixture, it is possible to more accurately identify the composition of the fuel mixture without being affected by the state of the fuel mixture or the surrounding environment of the fuel mixture.

Reference Signs List

1: composition identification system, 2: composition identification apparatus, 3: fuel unit, 4: heat engine, 5: moving object, 6: communication network, 7: another computer apparatus, 21: control unit, 22: main memory, 23: storage, 24: communication interface, 25: tank attaching unit, 26: sensor unit, 27: circuit unit.

## Claims

1. A composition identification program for identifying a composition of a mixture of three different liquids of a liquid A, a liquid B, and a liquid C,

    the composition identification program being configured to make a computer apparatus function as
    a composition identifier that identifies a composition of the mixture on a basis of a first characteristic value and a second characteristic value of the mixture, a first characteristic value and a second characteristic value unique to the liquid A, a first characteristic value and a second characteristic value unique to the liquid B, and a first characteristic value and a second characteristic value unique to the liquid C.

2. The composition identification program according to claim 1,

    the composition identification program being configured to make a computer apparatus function as
    a characteristic value identifier that identifies the first characteristic value and/or the second characteristic value unique to the liquid A, the first characteristic value and/or the second characteristic value unique to the liquid B, and/or the first characteristic value and/or the second characteristic value unique to the liquid C according to a temperature of the mixture.

3. The composition identification program according to claim 1 or 2, wherein,

    in a case where the first characteristic value of the mixture is P and the second characteristic value of the mixture is Q,
    the first characteristic value of the liquid A is $P_A$ and the second characteristic value of the liquid A is $Q_A$,
    the first characteristic value of the liquid B is $P_B$, and the second characteristic value of the liquid B is $Q_B$, and
    the first characteristic value of the liquid C is Pc and the second characteristic value of the liquid C is Qc,
    the composition identifier identifies a volume fraction X vol% of the liquid A, a volume fraction Y vol% of the liquid B, and/or a volume fraction Z vol% of the liquid C on a basis of a formula:

    [Mathematical formula 1]

$$X = 100 - Y - Z \quad \cdots (a)$$

$$Y = \frac{(P - P_A)(Q_C - Q_A) - (P_C - P_A)(Q - Q_A)}{(P_B - P_A)(Q_C - Q_A) - (P_C - P_A)(Q_B - Q_A)} \times 100 \quad \cdots (b)$$

$$Z = \frac{-(P - P_A)(Q_B - Q_A) + (P_B - P_A)(Q - Q_A)}{(P_B - P_A)(Q_C - Q_A) - (P_C - P_A)(Q_B - Q_A)} \times 100 \quad \cdots (c)$$

    and identifies the composition of the mixture.

4. The composition identification program according to claim 1 or 2, wherein the composition identifier identifies the composition of the mixture on a basis of a deviation amount between a volume fraction of the liquid A, the liquid B, and/or the liquid C calculated by a predetermined calculation formula and a volume fraction of the liquid A, the liquid B, and/or the liquid C in an actual mixture.

**5.** The composition identification program according to claim 1 or 2, wherein the first characteristic value is a dielectric constant or a parameter regarding the dielectric constant, and the second characteristic value is a thermal conductivity or a parameter regarding the thermal conductivity.

**6.** The composition identification program according to claim 1 or 2, wherein the liquid A is fossil fuel, the liquid B is hydrogenated vegetable oil, and the liquid C is bio-derived fuel.

**7.** The composition identification program according to claim 1 or 2,

the composition identification program being configured to make a computer apparatus function as
a composition memory that memorizes the identified composition of the mixture and/or a composition transmitter that transmits the identified composition of the mixture to another computer apparatus.

**8.** A composition identification apparatus for identifying a composition of a mixture of three different liquids of a liquid A, a liquid B, and a liquid C, the composition identification apparatus comprising
a composition identifier that identifies a composition of the mixture on a basis of a first characteristic value and a second characteristic value of the mixture, a first characteristic value and a second characteristic value unique to the liquid A, a first characteristic value and a second characteristic value unique to the liquid B, and a first characteristic value and a second characteristic value unique to the liquid C.

**9.** A composition identification apparatus for identifying a composition of a fuel mixture containing different types of fuels, the composition identification apparatus comprising
a composition identifier that identifies a composition of the fuel mixture on a basis of one or a plurality of characteristic values of the fuel mixture and one or a plurality of characteristic values unique to each of a plurality of the fuels contained in the fuel mixture.

**10.** The composition identification apparatus according to claim 9, further comprising
a composition memory that memorizes the identified composition of the fuel mixture and/or a composition transmitter that transmits the identified composition of the fuel mixture to another computer apparatus.

**11.** The composition identification apparatus according to claim 9 or 10, wherein the one or the plurality of characteristic values unique to each of the plurality of fuels are values identified according to a state of the fuel mixture or a surrounding environment of the fuel mixture.

**12.** A composition identification system comprising:

the composition identification apparatus according to claim 9 or 10;
a fuel unit in which a fuel mixture is present; and
one or a plurality of measurers capable of measuring one or a plurality of different characteristic values of the fuel mixture present in the fuel unit,
wherein the composition identifier identifies a composition of the fuel mixture on a basis of the one or the plurality of different characteristic values of the fuel mixture measured by the one or the plurality of measurers.

**13.** A moving object comprising:

the composition identification apparatus according to claim 9 or 10;
a fuel unit in which a fuel mixture is present;
one or a plurality of measurers capable of measuring one or a plurality of different characteristic values of the fuel mixture present in the fuel unit; and
a heat engine,
wherein the composition identifier identifies a composition of the fuel mixture on a basis of the one or the plurality of different characteristic values of the fuel mixture measured by the one or the plurality of measurers, and
wherein the moving object is driven by burning fuel supplied from the fuel unit in the heat engine.

FIG. 1

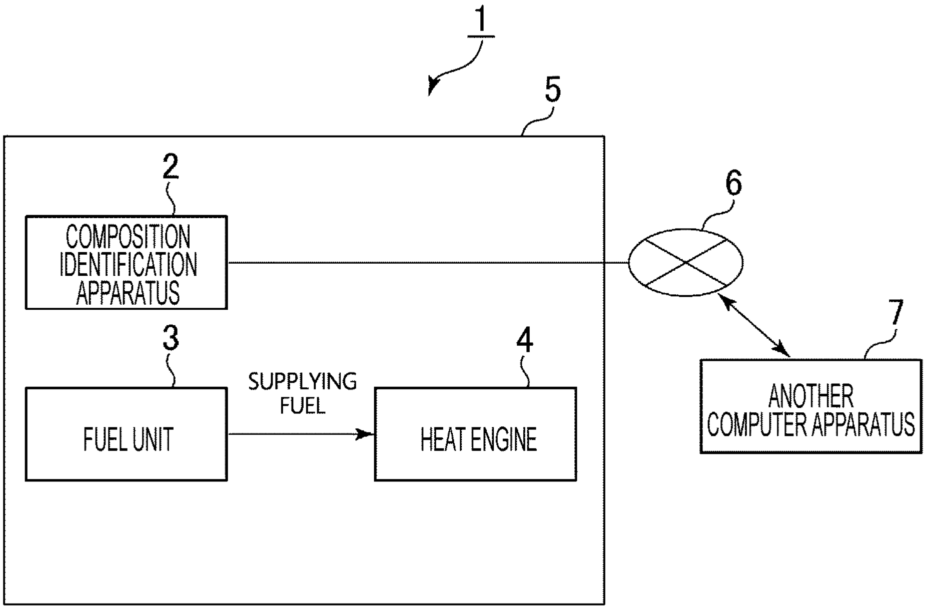
1
5
2
COMPOSITION IDENTIFICATION APPARATUS
3
FUEL UNIT
SUPPLYING FUEL
4
HEAT ENGINE
6
7
ANOTHER COMPUTER APPARATUS

FIG. 2

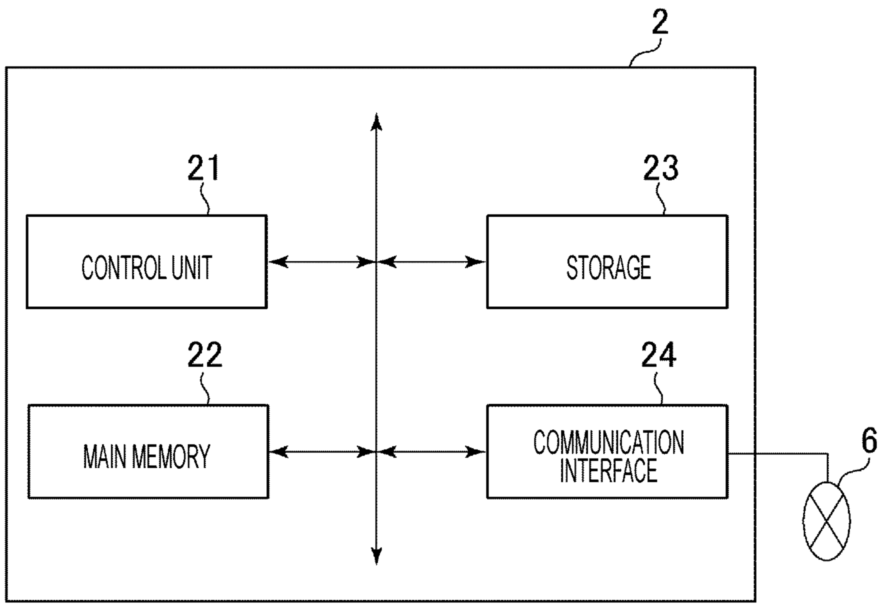
2
21
CONTROL UNIT
23
STORAGE
22
MAIN MEMORY
24
COMMUNICATION INTERFACE
6

FIG. 3

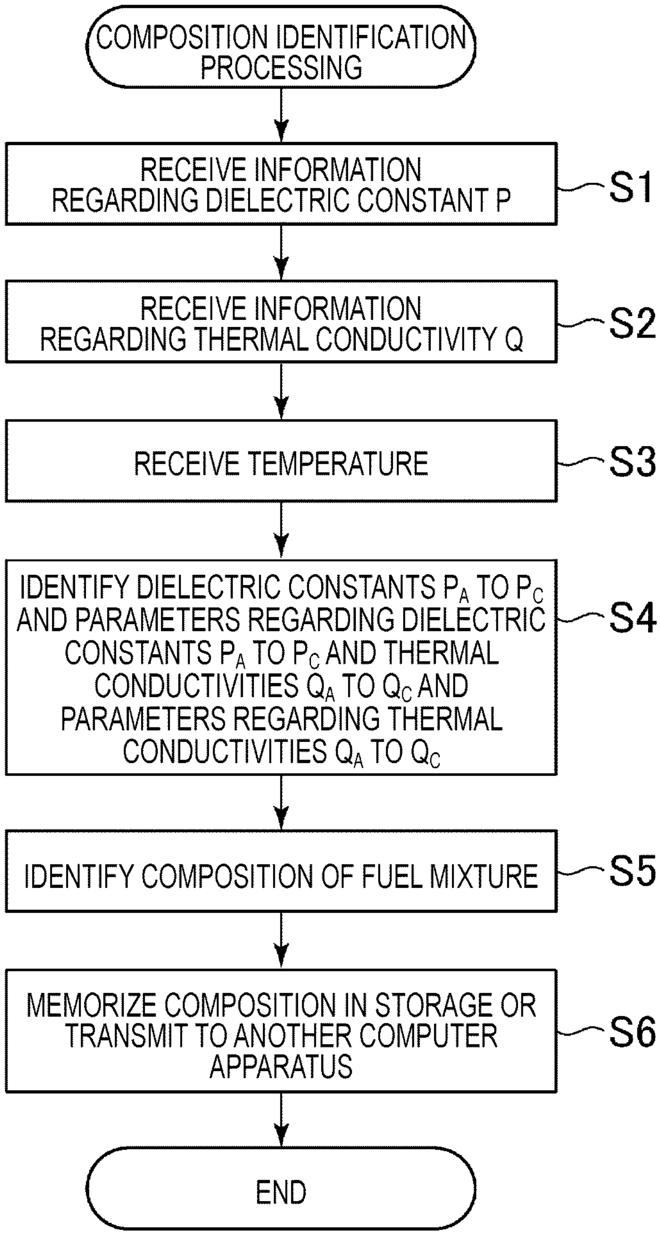
COMPOSITION IDENTIFICATION PROCESSING
RECEIVE INFORMATION REGARDING DIELECTRIC CONSTANT P
S1
RECEIVE INFORMATION REGARDING THERMAL CONDUCTIVITY Q
S2
RECEIVE TEMPERATURE
S3
IDENTIFY DIELECTRIC CONSTANTS $P_A$ TO $P_C$ AND PARAMETERS REGARDING DIELECTRIC CONSTANTS $P_A$ TO $P_C$ AND THERMAL CONDUCTIVITIES $Q_A$ TO $Q_C$ AND PARAMETERS REGARDING THERMAL CONDUCTIVITIES $Q_A$ TO $Q_C$
S4
IDENTIFY COMPOSITION OF FUEL MIXTURE
S5
MEMORIZE COMPOSITION IN STORAGE OR TRANSMIT TO ANOTHER COMPUTER APPARATUS
S6
END

FIG. 4

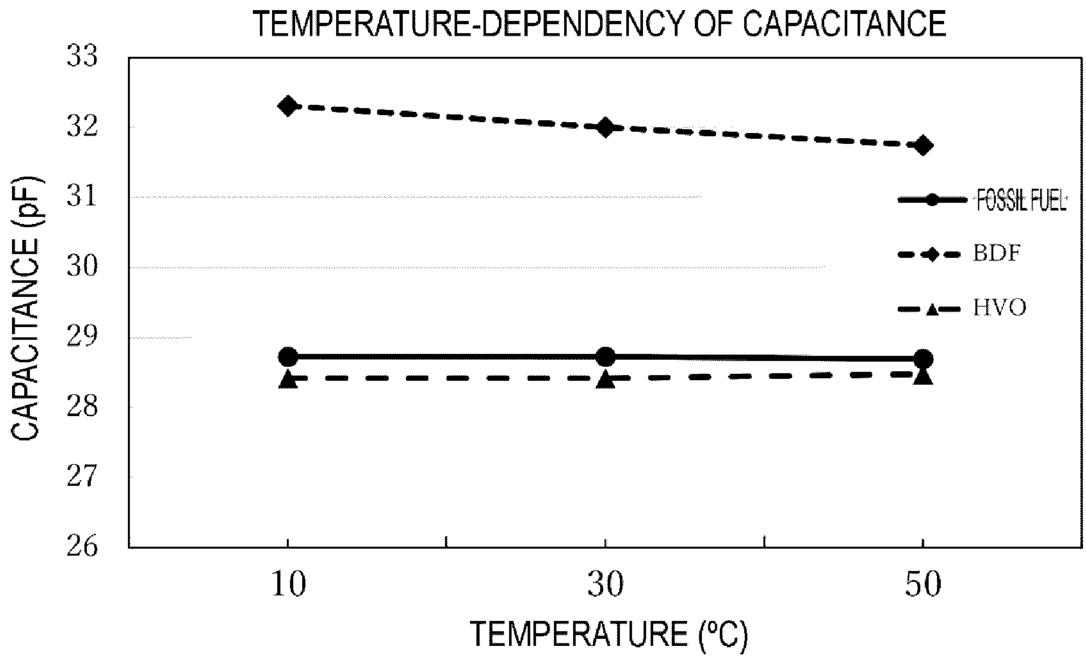
TEMPERATURE-DEPENDENCY OF CAPACITANCE
CAPACITANCE (pF)
33
32
31
30
29
28
27
26
FOSSIL FUEL
BDF
HVO
10
30
50
TEMPERATURE (ºC)

FIG. 5

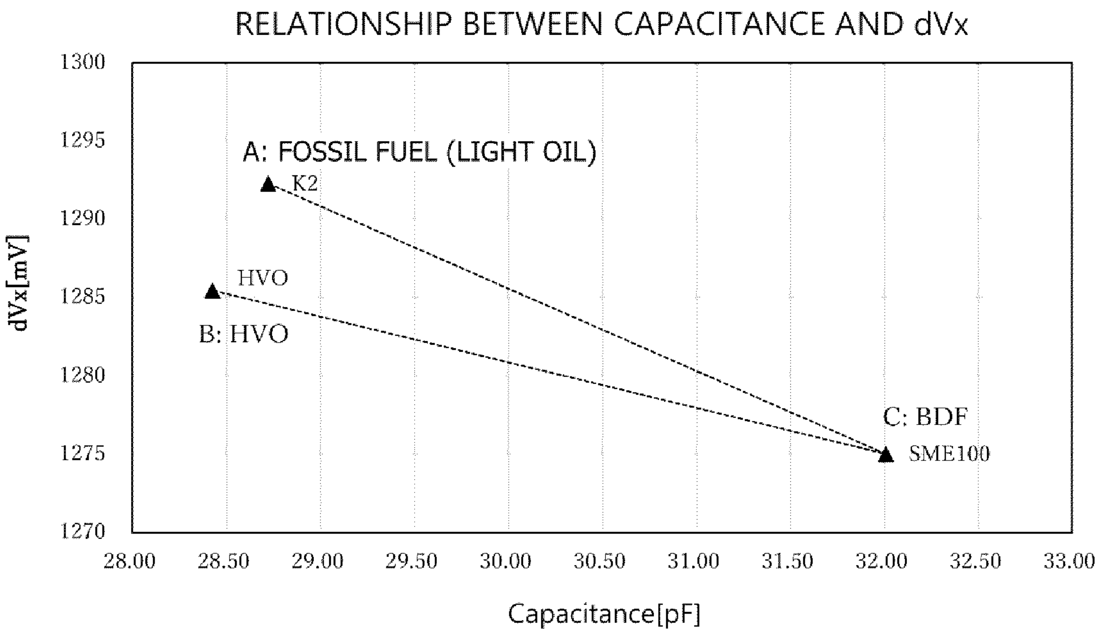
RELATIONSHIP BETWEEN CAPACITANCE AND dVx
dVx[mV]
1300
1295
1290
1285
1280
1275
1270
A: FOSSIL FUEL (LIGHT OIL)
K2
HVO
B: HVO
C: BDF
SME100
28.00
28.50
29.00
29.50
30.00
30.50
31.00
31.50
32.00
32.50
33.00
Capacitance[pF]

FIG. 6A

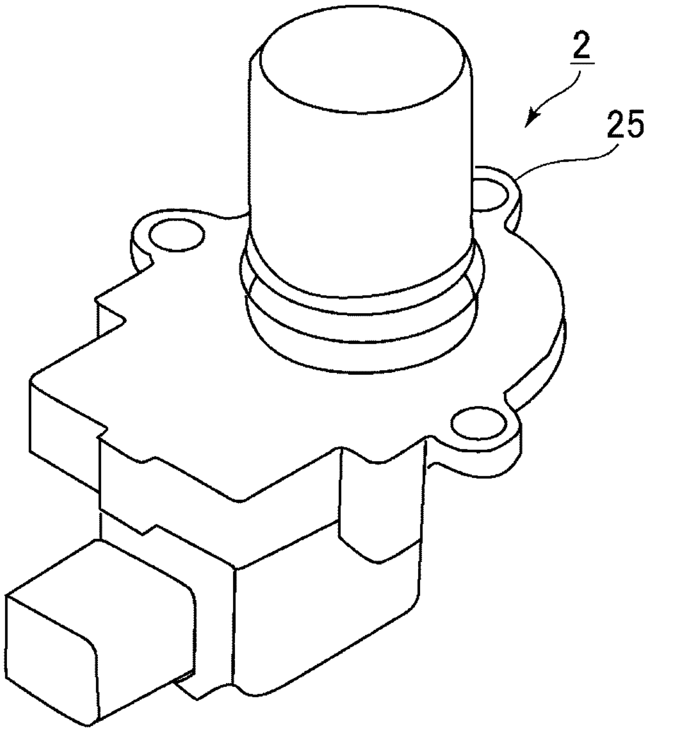


FIG. 6B

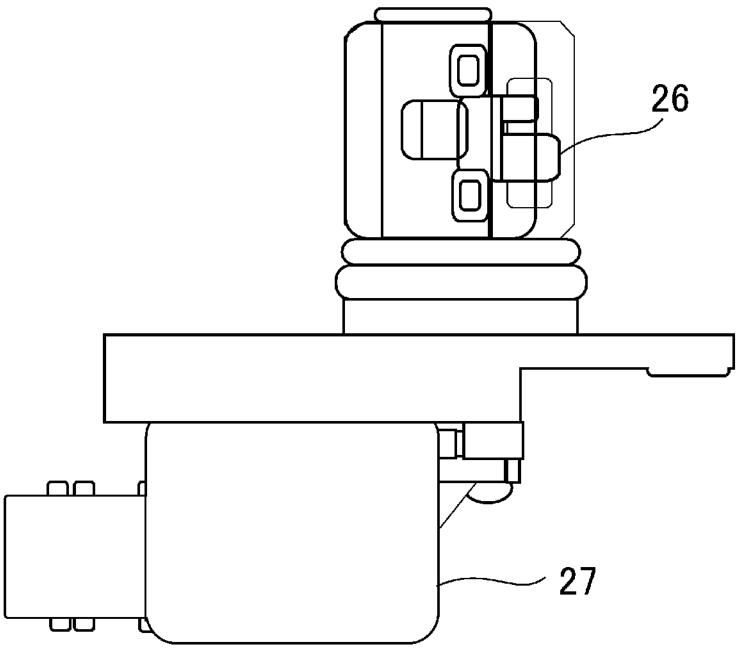

# FIG. 7

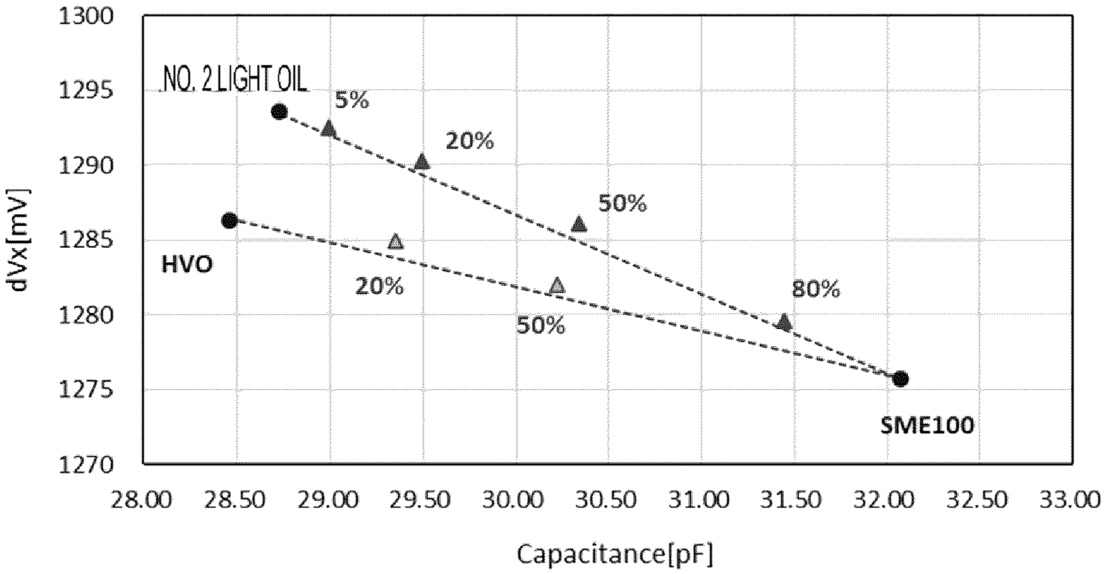
1300
1295
1290
1285
1280
1275
1270
dVx[mV]
NO. 2 LIGHT OIL
5%
20%
50%
80%
HVO
20%
50%
SME100
28.00
28.50
29.00
29.50
30.00
30.50
31.00
31.50
32.00
32.50
33.00
Capacitance[pF]

# FIG. 8

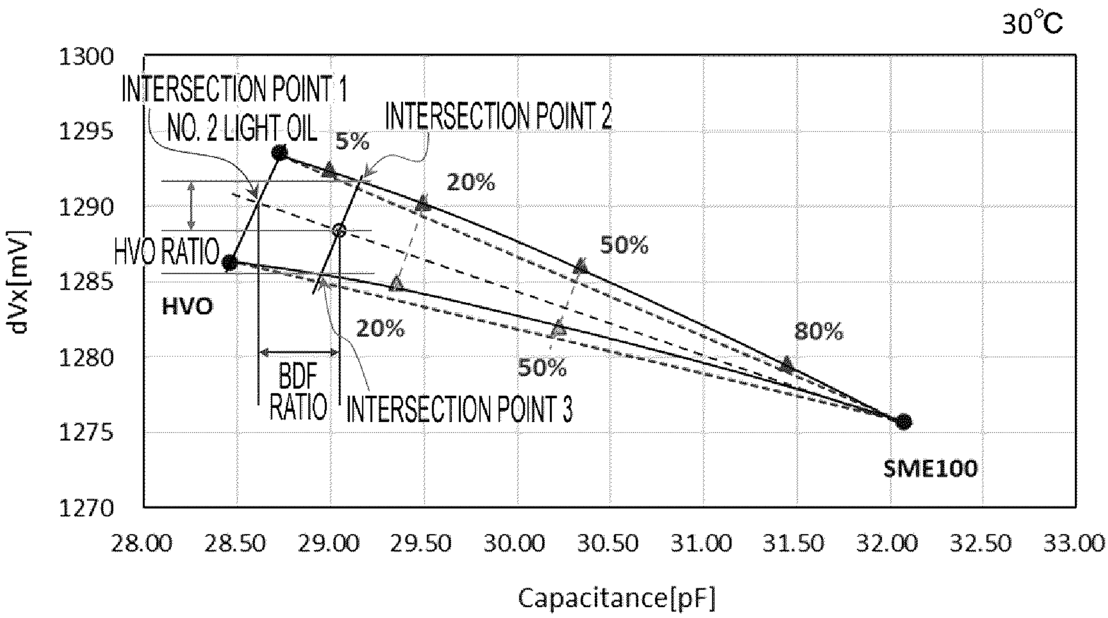
30℃
1300
1295
1290
1285
1280
1275
1270
dVx[mV]
INTERSECTION POINT 1
NO. 2 LIGHT OIL
5%
INTERSECTION POINT 2
20%
HVO RATIO
HVO
50%
20%
80%
50%
BDF
RATIO
INTERSECTION POINT 3
SME100
28.00
28.50
29.00
29.50
30.00
30.50
31.00
31.50
32.00
32.50
33.00
Capacitance[pF]

*FIG. 9*

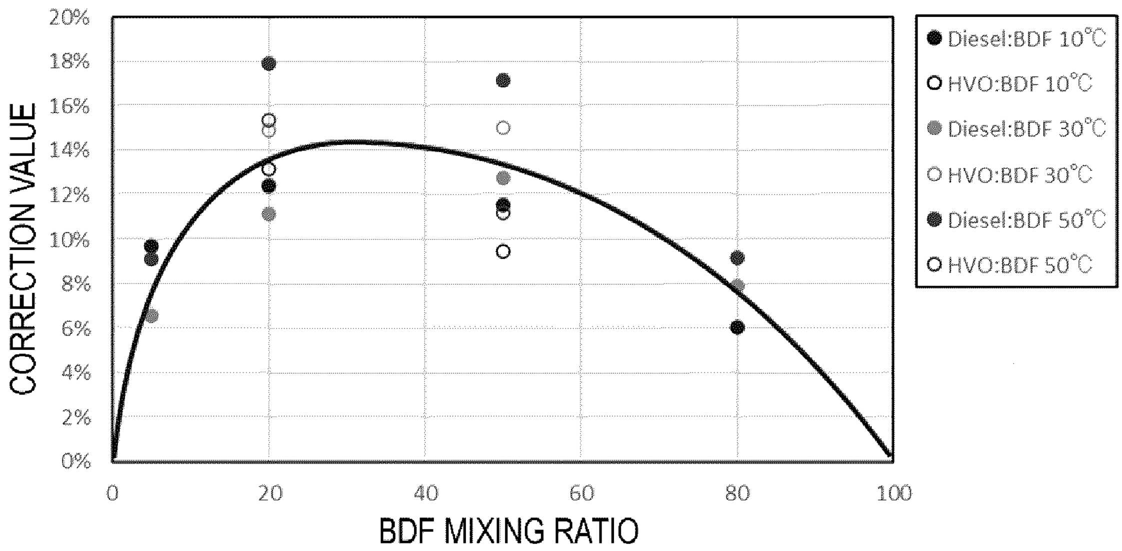
20%
18%
16%
14%
12%
10%
8%
6%
4%
2%
0%
CORRECTION VALUE
0
20
40
60
80
100
BDF MIXING RATIO
Diesel:BDF 10℃
HVO:BDF 10℃
Diesel:BDF 30℃
HVO:BDF 30℃
Diesel:BDF 50℃
HVO:BDF 50℃

*FIG. 10*

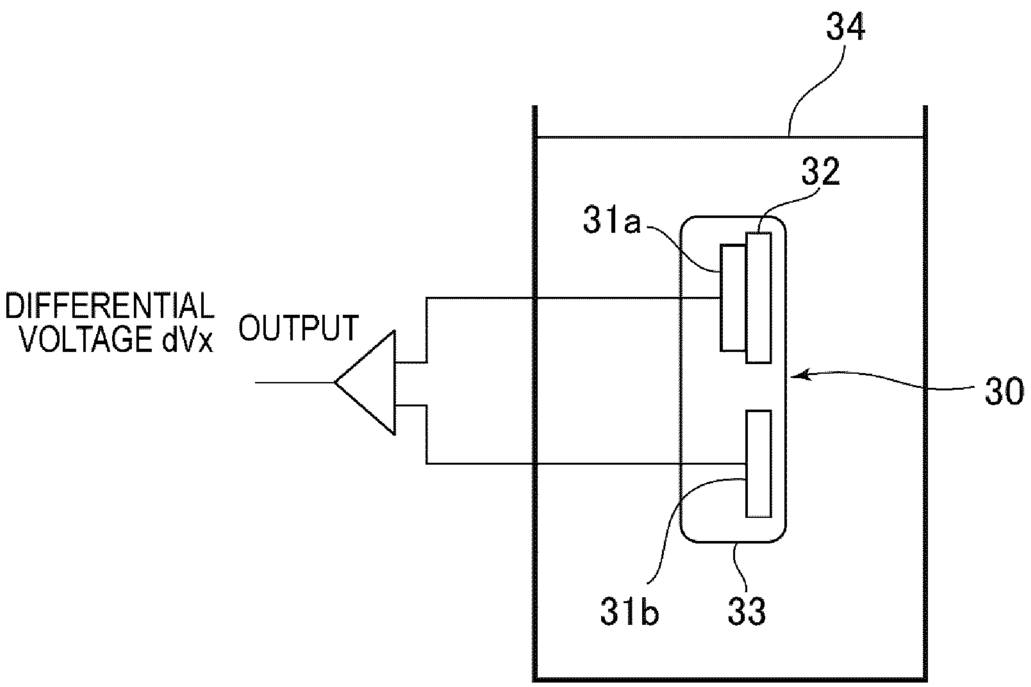
34
32
31a
DIFFERENTIAL VOLTAGE dVx
OUTPUT
30
31b
33

## INTERNATIONAL SEARCH REPORT

International application No.
**PCT/JP2023/016028**

**A. CLASSIFICATION OF SUBJECT MATTER**

***G01N 33/00***(2006.01)i; ***C10L 1/02***(2006.01)i; ***C10L 1/08***(2006.01)i; ***F02D 45/00***(2006.01)i; ***G01N 25/18***(2006.01)i; ***G01N 27/22***(2006.01)i; ***G01N 33/22***(2006.01)i
FI: G01N33/00 B; C10L1/02; F02D45/00 369; G01N27/22 B; G01N25/18 K; C10L1/08 ZAB; G01N33/22 B

According to International Patent Classification (IPC) or to both national classification and IPC

**B. FIELDS SEARCHED**

Minimum documentation searched (classification system followed by classification symbols)

G01N33/00; C10L1/02; C10L1/08; F02D45/00; G01N25/18; G01N27/22; G01N33/22

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 5-133926 A (NIPPONDENSO CO LTD) 28 May 1993 (1993-05-28) paragraphs [0001]-[0003] | 9-13 |
| A | JP 55-29725 A (ISHIKAWAJIMA HARIMA HEAVY IND) 03 March 1980 (1980-03-03) claim 1, p. 1, left column, line 15 to right column, line 3, p. 2, upper left column, line 10 to upper right column, line 15 | 1-13 |
| A | JP 2021-36225 A (OMRON TATEISI ELECTRONICS CO) 04 March 2021 (2021-03-04) claims 1, 15, 18, paragraphs [0004], [0005], [0009], [0037], [0053], [0122] | 1-13 |
| A | US 2002/0124630 A1 (JAESCHKE, Manfred) 12 September 2002 (2002-09-12) abstract, claims | 1-13 |
| A | US 5261270 A (GENERAL MOTORS CORPORATION) 16 November 1993 (1993-11-16) abstract, fig. 1, 2, claims | 1-13 |
| A | JP 7-306172 A (MITSUBISHI ELECTRIC CORP) 21 November 1995 (1995-11-21) claims | 1-13 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 July 2023** | **18 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/016028**

**C. DOCUMENTS CONSIDERED TO BE RELEVANT**

| Category* | Citation of document, with indication, where appropriate, of the relevant passages | Relevant to claim No. |
|---|---|---|
| A | JP 1-304348 A (NISSAN MOTOR CO LTD) 07 December 1989 (1989-12-07)<br>p. 1, right column, lines 1-5 | 1-13 |
| A | JP 2012-163508 A (MITSUBISHI ELECTRIC CORP) 30 August 2012 (2012-08-30)<br>paragraphs [0030], [0036] | 1-13 |
| A | US 5101367 A (AGAR CORPORATION, LTD.) 31 March 1992 (1992-03-31)<br>abstract | 1-13 |
| A | DE 102007054813 A1 (ROBERT BOSCH GMBH) 20 May 2009 (2009-05-20)<br>abstract, claims | 1-13 |
| A | US 2015/0168312 A1 (ROBERT BOSCH GMBH) 18 June 2015 (2015-06-18)<br>abstact, paragraphs [0004], [0005], [0008], claims | 1-13 |
| A | DE 102007025585 A1 (SIEMENS AG) 04 December 2008 (2008-12-04)<br>claims | 1-13 |
| A | WO 2017/018893 A1 (POLITECHNIKA WROCLAWSKA) 02 February 2017 (2017-02-02)<br>paragraph [0018], fig.1 | 1-13 |
| A | US 2010/0204928 A1 (LEPSCH, Fernando) 12 August 2010 (2010-08-12)<br>paragraph [0060] | 1-13 |
| A | JP 2020-531839 A (SAUDI ARABIAN OIL COMPANY) 05 November 2020 (2020-11-05)<br>claim 19, paragraphs [0005], [0036] | 1-13 |
| A | JP 6-347435 A (FORD MOTOR CO) 22 December 1994 (1994-12-22)<br>paragraph [0004] | 1-13 |
| A | JP 2009-531683 A (CONTINENTAL AUTOMOTIVE GMBH) 03 September 2009 (2009-09-03)<br>abstract, claims | 1-13 |

Form PCT/ISA/210 (second sheet) (January 2015)

**INTERNATIONAL SEARCH REPORT**
**Information on patent family members**

International application No.
**PCT/JP2023/016028**

| Patent document cited in search report | Publication date (day/month/year) | Patent family member(s) | Publication date (day/month/year) |
|---|---|---|---|
| JP 5-133926 A | 28 May 1993 | US 5270663 A<br>column 1, lines 5-55 | |
| JP 55-29725 A | 03 March 1980 | (Family: none) | |
| JP 2021-36225 A | 04 March 2021 | US 2021/0055239 A1<br>paragraphs [0004], [0005], [0009], [0037], [0075], [0148], claims<br>DE 102020121062 A<br>CN 112414489 A | |
| US 2002/0124630 A1 | 12 September 2002 | EP 939317 A2<br>DE 19808533 A | |
| US 5261270 A | 16 November 1993 | (Family: none) | |
| JP 7-306172 A | 21 November 1995 | US 5594163 A<br>claims<br>DE 19517390 A | |
| JP 1-304348 A | 07 December 1989 | DE 3917935 A | |
| JP 2012-163508 A | 30 August 2012 | (Family: none) | |
| US 5101367 A | 31 March 1992 | US 5503004 A<br>US 5263363 A<br>GB 2215061 A | |
| DE 102007054813 A1 | 20 May 2009 | (Family: none) | |
| US 2015/0168312 A1 | 18 June 2015 | WO 2013/064284 A1<br>DE 102011085490 A1<br>CN 104040325 A | |
| DE 102007025585 A1 | 04 December 2008 | (Family: none) | |
| WO 2017/018893 A1 | 02 February 2017 | (Family: none) | |
| US 2010/0204928 A1 | 12 August 2010 | WO 2009/009848 A1<br>EP 2171242 A1<br>CN 101802376 A | |
| JP 2020-531839 A | 05 November 2020 | WO 2019/040421 A1<br>paragraphs [0005], [0036], claim 19<br>EP 3673256 A1<br>KR 10-2020-0044042 A<br>CN 111194403 A | |
| JP 6-347435 A | 22 December 1994 | (Family: none) | |
| JP 2009-531683 A | 03 September 2009 | WO 2007/131814 A1<br>abstract, claims<br>US 2009/0088983 A1<br>EP 1951539 A1<br>CN 101370681 A<br>KR 10-2008-0085186 A | |

Form PCT/ISA/210 (patent family annex) (January 2015)